# EUROPÄISCHE PATENTSCHRIFT

(11) **EP 0 642 011 B1**
(45) Veröffentlichungstag und Bekanntmachung des Hinweises auf die Patenterteilung: **29.04.1998**
(21) Anmeldenummer: 93114006.5
(22) Anmeldetag: 02.09.1993
(51) Int. Cl.: G01N 7/02

(54) **Apparatur zur Bestimmung des Sauerstoffbedarfs**
Device for determining the oxygen demand
Appareil pour déterminer le besoin en oxygène

(43) Veröffentlichungstag der Anmeldung: 08.03.1995
(73) Patentinhaber: J.M. Voith GmbH, D-89522 Heidenheim (DE)
(72) Erfinder: Stieber,Michael, D-76131 Karlsruhe (DE)
(74) Vertreter: Weitzel, Wolfgang, Dr.-Ing. Patentanwalt

(56) Entgegenhaltungen:
- EP-A- 0 414 182
- DE-A- 2 146 127
- DE-A- 2 216 006
- DE-B- 1 256 919
- US-A- 3 740 320

## Beschreibung

Die Erfindung betrifft eine Apparatur zur Bestimmung des Sauerstoffbedarfs, insbesondere des biochemischen Sauerstoffbedarfs. Eine solche Apparatur ist bekannt aus DE 22 16 006 C1. Mit einer solchen Apparatur kann der biochemische Sauerstoffbedarf von belasteten Abwässern aber auch von natürlichen Gewässern überprüft werden, und zwar anhand von Proben, die diesen Gewässern bzw. Wassern entnommen werden. Diese Apparatur dient dazu, den Gehalt an biologisch abbaubaren Stoffen in gelöster und suspendierter Form an den Zu untersuchenden Wasserproben über den biochemischen Sauerstoffbedarf zu ermitteln. Das ist die Sauerstoffmenge in mg/1, die nötig ist, die organischen Stoffe eines Abwassers mit Hilfe von Mikroorganismen abzubauen.

Es ist nun vermehrt auch wünschenswert, Bodenuntersuchungen auf einer ähnlichen Basis durchzuführen.

Dementsprechend ist die Aufgabe der Erfindung, ein Verfahren und Einrichtungen anzugeben, die dies ermöglichen.

Diese Aufgabe wird durch die kennzeichnenden Merkmale des Patentanspruchs 1 sowie 5 gelöst.

Die Feststoffproben, die organische Stoffe enthalten, werden in ein Probengefäß, z. B. aus Glas oder Metall, eingebracht, in dem sie kontinuierlich über eine außenliegende Pumpe mit Wasser oder speziellen Mineralflüssigkeiten (Mineralwasser) durchspült werden können. Mit der Durchspülung wird im Flüssigmedium gelöster Sauerstoff in die Probe eingetragen und organische Stoffe können in Abhängigkeit von ihrer Wasserlöslichkeit und ihrer Adsorption am Feststoff in Lösung gehen. Die Durchspülung der Probe erfolgt von unten nach oben in einem geschlossenen Kreislauf, so daß sich zwischen gelöster und adsorbierter Phase ein Gleichgewicht einstellt. Der Eintrag von Sauerstoff in die Spülflüssigkeit erfolgt durch Diffusion über die Flüssigkeitsoberfläche. Damit diese Diffusion ausreichend schnell stattfinden kann, läuft die Spülflüssigkeit nach dem Austritt aus der Feststoffprobe in einem dünnen Film an dem inneren, direkt für die Aufnahme der Probe dienenden Probenraum, im allgemeinen ein getrennter Glasbehälter, herunter. Zusätzlich können durch Magnetantrieb über einen Magnetfisch Turbulenzen in der Spülflüssigkeit in dem am Boden des Probenbehälters befindlichen Spülflüssigkeitssammelraum erzeugt werden.

In der Probe vorhandene Mikroorganismen werden durch den Sauerstoffeintrag angeregt, einerseits biologisch aerob abbaubare Stoffe zu metabolisieren und andererseits sich zu vermehren. Die bei diesen Prozessen von den Mikroorganismen benötigte Sauerstoffmenge wird durch die Apparatur bestimmt. Dadurch kann das Abbauverhalten der organischen Kontaminanten der Feststoffprobe oder der bioverfügbare Anteil der Kontaminanten oder auch die Abbaukinetik bestimmt werden. Zum Beispiel muß das Abbauverhalten in der entsprechenden Feststoffprobe untersucht werden, selbst wenn bekannt ist, daß die vorhandenen organischen Stoffe prinzipiell biologisch abbaubar sind, da die Einbindung der Kontaminanten in die Matrix des Feststoffs deren Abbau entscheidend behindern bzw. stark begrenzen kann. Es kann somit auch festgestellt werden, ob vorhandene Mikroorganismen die Kontaminanten abbauen oder ob eine Beimpfung mit Spezialisten nötig ist.

Über die BSB-Kurven können die Abbaukinetiken der Kontaminanten berechnet werden und anhand von Parallelansätzen kann der Einfluß verschiedener Zuschlagstoffe, die für Wachstum und Vermehrung der Mikroorganismen nötig sind, untersucht und somit die optimale Zugabemenge bestimmt werden.

Nachfolgend wird die Erfindung anhand der Figuren der Zeichnung erläutert, die im einzelnen folgendes darstellen: Figur 1 ein allgemeines Schaltbild der Apparatur, Figur 2 und 3 jeweils prinzipmäßige Querschnitte durch das Probengefäß.

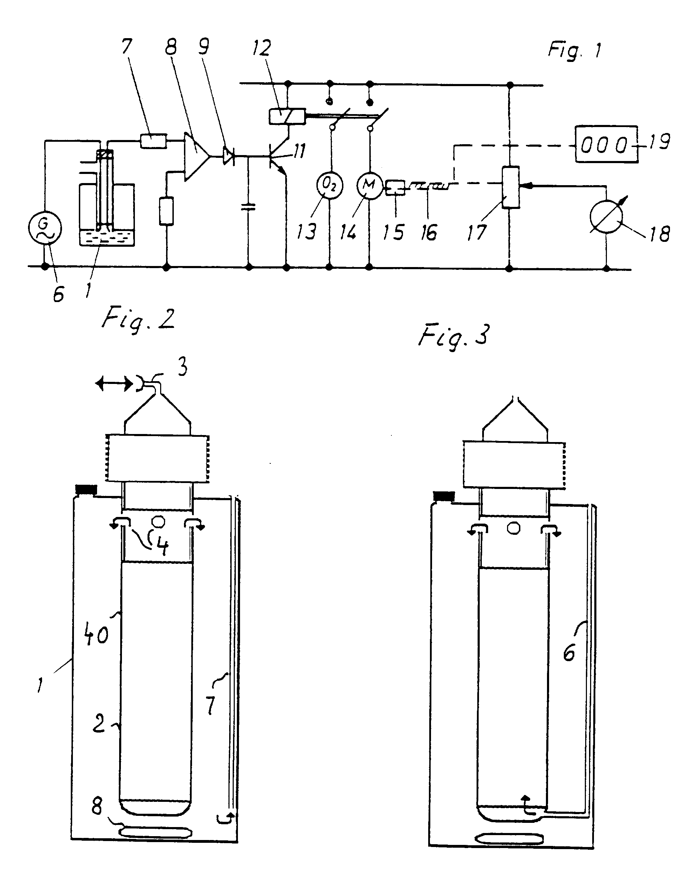

Gemäß Figur 1 ist ein geschlossenes System mit einem Probengefäß 1 und einem kontinuierlichen Sauerstofferzeuger 13 vorhanden, wobei letzterer vorzugsweise ein Elektrolysegerät ist.

Mit 6 ist ein Stromgenerator bezeichnet, der bei Schließen der Kontakte des Druckgebers - was im Fall von Unterdruck im geschlossenen System gegeben ist - über Widerstand 7, Verstärker 8 und Gleichrichter (Diode) 9 sowie über Transistor 11 und Schaltrelais 12 Spannung an den elektrolytischen Sauerstofferzeuger 13 legt. Gleichzeitig läuft dann ein Motor 14, der über Getriebe 15 und Rutschkupplung 16 ein Potentiometer 17 verstellt, das wiederum einen Punktdrucker 18 steuert. Diese Bauteile sind in Verbindung mit einer vorgegebenen Probemenge so ausgelegt, daß die Anzeige unmittelbar, z. B. in mg0₂/1 erfolgt. Dabei ist ein Endwert erreichbar, der über dem zu erwartenden Höchstwert des Sauerstoffbedarfs liegt. Ebenfalls mit dem Motor 14 verbunden ist ein Digitalzählwerk 19, das den jeweils erreichten Gesamtwert anzeigt.

In Figur 2 ist das vorzugsweise aus Glas bestehende Probengefäß 1 mit einem inneren Glasbehälter 2 dargestellt, welcher die eigentliche Feststoffprobe, z. B. eine Bodenprobe, aufnimmt. Man erkennt unten am Boden des Gefäßes 1 einen Magnetfisch 8, der über einen Magnetantrieb ein Umrühren der dort sich sammelnden Flüssigkeit bewirkt. Es ist ferner eine Zufuhrleitung 6 sowie eine Abführleitung 7 für die Flüssigkeit zu erkennen. Sie gehören zu einem von einer Pumpe betriebenen Kreislauf einer Spülflüssigkeit, z. B. Wasser oder insbesondere auch mineralisiertes Wasser oder eine sonstige ähnliche Flüssigkeit, die von unten nach oben die Bodenprobe 40 durchströmt. Nach Durchströmen dieser Probe tritt die Flüssigkeit durch die Öffnungen 4 wieder aus und läuft als Film entlang des Behälters 2 nach unten, so daß eine starke Sauerstoffaufnahme hier erfolgen kann. Die Zufuhr des Sauerstoffs erfolgt über Stutzen 3 von dem Sauerstofferzeuger 13 her, und zwar wie bereits erwähnt, gesteuert von dem Druckgeber abhängig von dem Unterdruck im geschlossenen System, je nach dem, wieviel Sauerstoff von den in der Probe enthaltenen Mikroorganismen verbraucht wird.

Es ist andererseits auch ein Verfahren zur Bestimmung des BSB anwendbar, bei dem die zum Kompensieren eines in einem abgeschlossenen System entstehenden Unterdruckes erforderliche, aus einem Vorrat dem System zugeführte Sauerstoffmenge dadurch gemessen wird, daß diese Sauerstoffmenge jeweils bei Erreichen einer bestimmten Höhe des Unterdruckes zeitlich diskontinuierlich während einer von der Sauerstoffverbrauchs-Geschwindigkeit abhängigen Dauer zugeführt wird, und zwar mit pro Zeiteinheit konstanter Menge, und daß die zugeführte Sauerstoffmenge durch Summierung der Zufuhrdauer ermittelt wird.

Die Messung des Sauerstoffverbrauches kann also analog oder digital erfolgen, wobei die von einem Rechner erfaßten Meßdaten über spezielle Programme ausgewertet werden können. Die Darstellung der Meßergebnisse ist sowohl mengenbezogen als auch zeitbezogen möglich. Im übrigen sind die Messungen allgemein sich über mehrere Tage erstreckende Langzeitmessungen.

## Patentansprüche

1. Verfahren zur Bestimmung des biochemischen Sauerstoffbedarfs bei Oxydationsvorgängen, bei dem in einem geschlossenen System eine Probe (P) des zu untersuchenden Substrats eingebracht wird, durch die mit der Probe eingebrachten Mikroorganismen wird Sauerstoff im geschlossenen System verbraucht, wodurch ein Unterdruck entsteht, welcher mit Hilfe eines Sauerstofferzeugers ausgeglichen wird und die für den Ausgleich nötige erzeugte und gemessene Sauerstoffmenge den biochemischen Sauerstoffbedarf der Probe darstellt, wobei im allgemeinen ein Adsorbateur für im System erzeugtes CO₂ vorhanden ist, dadurch gekennzeichnet, daß für die Untersuchung von Feststoffproben diese von unten nach oben von mit Sauerstoff angereichertem Spülwasser in konstanter Menge durchströmt wird.

2. Verfahren nach Anspruch 1, dadurch gekennzeichnet, daß die Spülflüssigkeit in konstanter Menge im Kreislauf geführt wird.

3. Verfahren nach Anspruch 1 oder 2, dadurch gekennzeichnet, daß die von der Probe (P) abgeleitete Flüssigkeitsmenge als - ausgebreiteter - Film einem in der Nähe des oder auf dem unteren Probenbereichsniveau gelegenen Sammelraum zugeleitet wird.

4. Verfahren nach einem der Ansprüche 1-3, dadurch gekennzeichnet, daß die Anreicherung der Spülflüssigkeit mit Sauerstoff oberhalb eines unteren Grenzwerts erfolgt, der oberhalb des maximalen biochemischen Sauerstoffbedarfs liegt.

5. Einrichtung zur Durchführung des Verfahrens, bei welchem ein Probengefäß (1), in dem der Oxidationsvorgang abläuft und eine Vorrichtung zum druckabhängigen Auslösen der Zufuhr eines Sauerstoffstromes aus einem Vorrat oder einem Sauerstofferzeuger in einem geschlossenen System vorhanden ist, dadurch gekennzeichnet, daß das Probengefäß (1) einen die Feststoffprobe (P) enthaltenden, inneren Behälter (2) sowie eine Spülwasserzuleitung (6) und eine Spülwasserableitung (7) aufweist.

6. Einrichtung nach Anspruch 5, dadurch gekennzeichnet, daß der Boden des Probengefäßes (1) als Sammelraum für das Spülwasser dient, dem dieses nach Durchströmen der Probe als Film entlang der Wandung des inneren Behälters (2) zuläuft.

## Claims

1. A method of determining the biochemical oxygen requirement in oxidation processes, in which a specimen (P) of the substrate to be examined is introduced into a closed system, oxygen being consumed in the closed system by microorganisms introduced together with the specimen, so that a negative pressure is created which is compensated by means of an oxygen generator, the quantity of oxygen produced and required for compensation and measured constituting the biochemical oxygen requirement of the specimen, an adsorber for C02 generated in the system being generally present, characterised in that for examining specimens of solids, a constant quantity of oxygen-enriched flushing water flows through the specimen from the bottom upwards.

2. A method according to claim 1, characterised in that the flushing fluid is circulated in a cycle and in a constant quantity.

3. A method according to claim 1 or 2, characterised in that the quantity of fluid derived from the specimen (P) and in the form of a spread-out film is fed to a collecting space situated in the vicinity of or at the lower level of the specimen area.

4. A method according to one of claims 1 to 3, characterised in that enrichment of the flushing liquid with oxygen takes place above a lower limit value which is above the maximum biochemical oxygen requirement.

5. An apparatus for carrying out the method, in which a specimen vessel (1) in which the oxidation process takes place and a device for the pressure dependent release of the feed of a flow of oxygen from a stock or an oxygen generator is provided in a closed system, characterised in that the specimen Vessel (1) comprises an inner container (2) containing the solids specimen (P) as well as a flushing water supply line (6) and a flushing water discharge line (7).

6. An apparatus according to claim 5, characterised in that the bottom of the specimen vessel (1) serves as a space for collecting the flushing water, this latter flowing to it after passing through the specimen as a film along the walls of the inner container (2).

## Revendications

1. Procédé de détermination du besoin biochimique en oxygène dans des processus d'oxydation, dans lequel un échantillon (**P**) du substrat à examiner est introduit dans un système fermé, les micro-organismes introduits par l'échantillon provoquent une consommation d'oxygène dans le système fermé, ce qui conduit à une pression négative, qui est compensée à l'aide d'un producteur d'oxygène et la quantité d'oxygène, produite et mesurée, nécessaire à la compensation, représente le besoin biochimique en oxygène de l'échantillon, un adsorbeur du CO₂ produit dans le système étant généralement présent, caractérisé en ce que, pour l'examen d'un échantillon solide, celui-ci est traversé de bas en haut en quantité constante par de l'eau de rinçage enrichie en oxygène.

2. Procédé selon la revendication 1, caractérisé en ce que le liquide de rinçage est introduit en quantité constante dans le circuit.

3. Procédé selon la revendication 1 ou 2, caractérisé en ce que la quantité de liquide évacuée de l'échantillon (**P**) est envoyée sous la forme d'un film - étendu - à une chambre de collecte située au niveau inférieur de la zone échantillon ou à proximité de ce niveau.

4. Procédé selon l'une des revendications 1 à 3, caractérisé en ce que l'enrichissement du liquide de rinçage en oxygène se fait au-dessus d'un seuil inférieur qui est supérieur au besoin biochimique maximal en oxygène.

5. Dispositif pour appliquer le procédé, dans lequel un récipient à échantillon (**1**), dans lequel se déroule le processus d'oxydation, et un dispositif de déclenchement en fonction de la pression de l'arrivée d'un courant d'oxygène depuis un réservoir ou un producteur d'oxygène sont présents dans un système fermé, caractérisé en ce que le récipient à échantillon (**1**) présente un récipient intérieur (**2**) contenant l'échantillon solide (**P**), ainsi qu'une conduite d'eau de rinçage (**6**) et une évacuation d'eau de rinçage (**7**).

6. Dispositif selon la revendication 5, caractérisé en ce que le fond du récipient à échantillon (**1**) sert de chambre de collecte pour l'eau de rinçage, vers laquelle celle-ci s'écoule sous la forme d'un film le long de la paroi du récipient intérieur (**2**) après avoir traversé l'échantillon.
